# EUROPEAN PATENT SPECIFICATION

(11) **EP 0 961 603 B1**
(45) Date of publication and mention of the grant of the patent: **25.06.2003**
(21) Application number: 96945073.3
(22) Date of filing: 19.12.1996
(51) Int. Cl.: A61F 13/15

(54) **ABSORBENT ARTICLES HAVING A SEPARATING MEANS**
ABSORBIERENDE ARTIKEL MIT TRENNMITTEL
ARTICLES ABSORBENTS COMPORTANT UN MOYEN DE SEPARATION

(30) Priority: 28.12.1995 EP 95120653
(43) Date of publication of application: 08.12.1999
(73) Proprietor: THE PROCTER & GAMBLE COMPANY, Cincinnati, Ohio 45202 (US)
(72) Inventor: BEWICK-SONNTAG, Christopher, Philip, I-65125 Pescara (IT); VEGLIO, Paolo, I-65100 Pescara (IT)
(74) Representative: Kohol, Sonia
(86) International application number: US9620712
(87) International publication number: WO97024097

(56) References cited:
- EP-A- 0 174 152
- EP-A- 0 203 822
- EP-A- 0 293 482
- EP-A- 0 710 472
- WO-A-92/07535
- WO-A-94/16658
- US-A- 4 591 523
- US-A- 5 346 487
- US-A- 5 527 303

## Description

### Field of the Invention

The present invention relates to absorbent articles in particular sanitary napkins having a breathable backsheet, which exhibit reduced wet through onto the users garments.

### Background of the Invention

The primary consumer needs which underlie development in the absorbent article field, in particular catamenials is the provision of products providing both a high protection and comfort level.

One highly desirable means for improving the comfort of absorbent articles is the use of so called 'breathable backsheets'. Breathable backsheets may typically comprise either a microporous film or an apertured formed film having directional fluid transfer as disclosed in for example US 4 591 523. Both types of breathable backsheets are vapour permeable, allowing gaseous exchange with the environment. This thereby allows for the evaporation of a portion of the fluid stored in the core and increases the circulation of air within the absorbent article. This is particularly beneficial as it reduces the sticky feeling experienced by many wearers during use, particularly over extended periods of time.

However, the main drawback associated with the use of breathable backsheets in absorbent articles is the increased probability of leakage, commonly referred to as wet through, onto the users garment. Although the breathable backsheets in principle only allow the transfer of materials in the gaseous state, physical mechanisms such as extrusion, diffusion and capillary action may still occur and result in the transfer of the fluids from the absorbent core through the backsheet and onto the users garments. In particular, these mechanisms become more dominant if the product is utilised during physical exertion, for heavy discharge loads or over extended periods of time. Thus, whilst the incorporation of breathable backsheets in absorbent articles is highly desirable from a comfort standpoint, they result in an unacceptable level of failure with regard to protection, especially under stressed conditions.

The problem of wet through onto users garments due to the incorporation of such breathable backsheets in absorbent articles has been recognised in the art. However attempts to solve the problem have mainly resided in the use of multiple layer backsheets such as those illustrated in US 4 341 216. Similarly document EP 0 710 471 discloses a breathable backsheet for disposable absorbent articles comprising an outer layer of a gas permeable, hydrophobic, polymeric fibrous fabric and an inner layer comprising an apertured formed film having directional fluid transport. Also document EP 0 710 472 discloses breathable absorbent articles comprising a breathable backsheet consisting of at least two breathable layers which are unattached to one another over the core area.

Document EP 0 203 822 discloses an absorbent device having a backsheet comprising a combination of two layers. The first laye comprises a liquid - impervious polymeric film and the second layer is a polymeric film with a three-dimensional pattern.

However, none of the above solutions have proved fully satisfactory in providing a satisfactory solution to the problem of backsheet wet through. Furthermore, the problem of wet through is further accentuated in the case of so called thin products, which are again desirable from a comfort standpoint. Thus, there exists a dichotomy in the means available to provide increased comfort absorbent products, such that thin breathable products cannot provide the desired level of protection.

As a result, there exists a need to provide an absorbent article which offers improved comfort by the employment of a breathable backsheet and having a reduced thickness which maintains the required level of protection.

It has now been found that breathable backsheets may be utilised in sanitary napkins having reduced thickness, thereby providing a product having both a high level of protection and comfort by the utilisation of a separating means between any adjacent layers below and including the core. It is believed that the separation of the layers, particularly the core from the breathable backsheet thereby minimising the contact between these layers hinders the movement of fluid through the backsheet onto the users garments thereby preventing wet through.

Separating means have been previously disclosed in the context of absorbent articles, such as for example EPO 555 341. However, this document does not disclose breathable backsheets or recognise the problems associated with the use of such breathable backsheets.

### Summary of the Invention

The present invention is a disposable absorbent article comprising, a liquid pervious topsheet, an absorbent core and a breathable backsheet. The absorbent core is positioned intermediate the topsheet and the backsheet, and each comprise at least one layer, the core comprising a fluid storage layer and the backsheet comprising an outer layer. Each of the layers has a wearer facing surface and a garment facing surface and each of these wearer facing surfaces forms a common interface with an adjacent garment facing surface of an adjacent layer. The article has a backsheet portion extending from the garment facing surface of the fluid storage layer to the garment facing surface of the outer layer.

At least one garment facing surface in the backsheet portion is separated from an adjacent wearer facing surface by a gas-permeable separating means in the form of a layer such that not more than 50% of the surface of the common interface is in direct contact.

The present invention is characterised in that said outer layer of said backsheet is a microporous two-dimensional polymeric apertured film, the apertures having average diameters of from 150 µm to 5 µm.

### Detailed Description of the Invention

The present invention relates to absorbent disposable articles such as sanitary napkins, baby diapers, incontinence products and panty liners. Typically such products comprise a liquid pervious topsheet, a backsheet and an absorbent core intermediate said topsheet and said backsheet. The topsheet, core and backsheet each comprise at least one layer, each of these layers having a wearer facing surface and a garment facing surface. Each of the wearer facing surfaces forms a common interface with an adjacent garment facing surface of an adjacent layer, herein after referred to as common interface. The common interface extends to the same area as those portions of the respective garment and wearer facing surfaces which are coextensive.

The absorbent article has a backsheet portion extending from the garment facing surface of the storage layer to the garment facing surface of the outer layer of the backsheet. The backsheet portion is herein after referred to as any layer below and including the core.

According to a preferred embodiment of the present invention the absorbent article comprises sideflaps, side wrapping elements or wings.

### Separating means

According to the present invention the absorbent article comprises as an essential feature a separating means. The term separating means as used herein refers to any device which separates the common interface of two adjacent surfaces as defined above. Common interfaces which may be thus separated include the garment facing surface of the fluid storage layer from the wearer facing surface of the backsheet and any pair of adjacent backsheet layers and any combinations thereof. According to the present invention said garment facing surface and said wearer facing surface are separated from one another such that not more than 50%, preferably not more than 45%, more preferably not more than 40%, most preferably not more than 30% of the surface of the common interface is in direct contact. It is believed that the separation of at least two adjacent layers below and including the core significantly reduces the occurrence of wet through onto the undergarment which would normally occur with a breathable backsheet.

In addition the absorbent article and similarly each layer of said absorbent article has a side portion extending from the periphery of the absorbent article to the centre or central portion of the absorbent article, said central portion being defined as an area which typically receives at least 75%, of the fluid discharge. The central portion of the absorbent article is thus under the most stress and where the probability of wet through failure is greatest. Therefore in a preferred embodiment direct contact at the common interface surface at this central portion should be minimised and is preferably such that not more than 50%, preferably not more than 40%, most preferably not more than 30% of said central portion of said surface of said common interface is in direct contact.

According to the present invention said separating means may be any means whereby a common interface of two adjacent layers in the backsheet portion of the article are separated from one another and thus include those described in EPO 555 341.

The separating means of the present invention has a longitudinal centre line and transverse centre line which may be used to describe the position and orientation of the separating means under the core of the absorbent article. The separating means may be placed in various locations under the absorbent core. For instance the separating means may be centred relative to the other components of the absorbent article so that the longitudinal and transverse lines of the separating means coincide with the principal longitudinal and transverse centrelines of the absorbent article. Alternatively, the separating means may be positioned so that it is not centred on the absorbent article such that the longitudinal and transverse centrelines of the separating means and the absorbent article are offset.

The separating means may have any configuration provided that it separates two adjacent layers as described herein above. Hence, the separating means may be symmetrical or unsymmetrical about its principal longitudinal and transverse centrelines.

The configuration of the separating means may be described in terms of its longitudinal and transverse dimensions. Typically, the longitudinal dimensions of the separating means are greater than the transverse dimensions. The separating means can extend up to 100% of the length and of the width of any of the surfaces of the layers which it separates. In addition, in order to minimise the effect on the overall thickness of the absorbent article the separating means should be thin and preferably have a thickness or depth less than 1mm preferably less than 0.5mm.

The separating means may be comprised of one element or a number of elements provided that separation between the layers is achieved. Preferably, the separating means comprises one element. Similarly, the separating means may be comprised of the same piece and type of material or may be comprised of separate pieces or types of material. Preferably the separating means is comprised of one piece of material which is unitary.

According to the present invention the separating means may be made of any material provided that it does not substantially hinder the transfer of gaseous materials between the layers which it separates. Suitable materials for the separating means include polymeric materials such as polyethylene, polypropylene and adhesive materials which can be chosen depending on the configuration of the separating means and the degree of stability required.

In one preferred embodiment of the present invention said separating means comprises a lattice which is centred with the absorbent article and extends to the periphery of the absorbent article. The lattice may have any dimension depending on the configuration of the separating means but preferably has a net-like configuration. Typically the lattice comprises openings having an average diameter of at least 100 micrometers, preferably at least 150 micrometers, most preferably at least 200 micrometers. Materials suitable for use as a lattice separating means include adhesives and polypropylene.

In another preferred embodiment of the present invention said separating means is comprised of a sheet material. The sheet material may be planar, folded or pleated as described for example in EPO 555 341. Particularly suitable material includes an apertured planar polymer material or an apertured formed polymeric film, the apertures having an average diameter of at least 100 micrometers, preferably at least 150 micrometers, most preferably at least 200 micrometers.

### Backsheet

The absorbent articles according to the present invention comprises a breathable backsheet. The backsheet primarily prevents the extrudes absorbed and contained in the absorbent structure from wetting articles that contact the absorbent product such as underpants, pants, pyjamas and undergarments. In addition however, the breathable backsheet of the present invention permits the transfer of both vapour and air through it and thus allows the circulation of air into and out of the backsheet.

According to the present invention the breathable backsheet comprises at least one gas permeable layer. Suitable gas permeable layers include 2 dimensional, planar micro and macro-porous films, macroscopically expanded films and formed apertured films. According to the present invention the apertures in said layer may be of any configuration, but are preferably spherical or oblong and may also be of varying dimensions. Typically, said layers have apertures having average diameters of from 150 micrometers to 5 micrometers, preferably from 120 micrometers to 10 micrometers, most preferably from 90 micrometers to 15 micrometers. The apertures preferably are evenly distributed across the entire surface of the layer, however layers having only certain regions of the surface having apertures is also envisioned.

Suitable 2 dimensional planar layers of the backsheet may be made of any material known in the art, but are preferably manufactured from commonly available polymeric materials. Suitable materials are for example Gortex (TM) or Sympatex (TM) type materials well known in the art for there application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures all lie within the plane and do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 4 637 819 and US 4 591 523.

According to the present invention the backsheet may comprise in addition to said gas permeable layer additional backsheet layers. Said additional layers are located adjacent and below said gas permeable layer towards the garment facing surface of the outer layer. The additional layers may be of any material preferably such that they do not reduce the gas permeability of the backsheet.

The backsheet typically extends across the whole of the absorbent structure and can extend into and form part of or all sideflaps, side wrapping elements or wings.

According to the present invention the absorbent article can be used beneficially in the context of sanitary napkins, panty liners, incontinence articles and diapers. However, sanitary napkins and panty liners are particularly susceptible to the present invention. The disposable article may thus have all those features and parts which are typical for products in the context of their intended use. In addition to the components previously described they typically comprise a topsheet and absorbent core.

### Absorbent core

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent core may have any thickness depending on the end use envisioned. In a preferred embodiment of the present invention wherein the absorbent article is a sanitary napkin or a panty liner, the core may have a thickness of from 15mm to 1mm, preferably from 10mm to 1mm, most preferably from 7mm to 1mm.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents. Active carbon coated with or in addition to other odor control agents, in particular suitable zeolite or day materials, are optionally incorporated in the absorbent structure. These components can be incorporated in any desired form but often are included as discrete particles.

### The topsheet

The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as non woven fabrics, films or combinations of both. In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 246, US 4 343 314 and US 4 591 523.

The topsheet typically extends across the whole of the absorbent structure and can extend into and form part of or all of the preferred sideflaps, side wrapping elements or wings.

According to the present invention the absorbent article is constructed by joining the various elements such as topsheet, backsheet and absorbent core by any means well known in the art. For example the backsheet and/or topsheet may be joined to the absorbent core or to each other by a uniform continuous layer of adhesive, a patterned layer of adhesive, or any array of separate lines, spirals or spots of adhesive. Alternatively, the elements may be joined by heat bonds, pressure bonds, ultra sonic bonds, dynamic mechanical bonds or any other suitable joining means known in the art and any combination thereof.

### Examples

### Undergarment Wet-through Evaluation Test

An undergarment wet-through test was carried out on sanitary napkins according to the present invention as detailed below to evaluate the resistance of the backsheet to transmission of bodily discharges.

### Method:

This is a test that is performed under realistic in-use conditions. A group of at least 20, for example women menstruating, are given at least 5 sanitary napkins and an equal number of cotton panties. They are instructed to wear the sanitary napkin with the supplied panties as they would normally wear such articles. Following usage the panty is removed with the used napkin still attached and transported to the laboratory for visual assessment of the used napkin and the panty.

The returned napkins are inspected for through backsheet wet-through. In the case of an sanitary napkin worn during menstruation this consists of quantifying the number of panties that feature a reddish blemish greater than 0.25 mm². For each napkin that has wet-through the breathable backsheet, the quantity of menses, the location of wet-through and magnitude are recorded.

### Examples:

Each test sample was prepared under identical conditions in all regards except for the specific aspect detailed in the example description below. All test samples were machine made "Always Ultra Normal" sanitary napkins {manufactured by Procter & Gamble, Pescara Technical Centre SpA, Italy with the modification detailed} according to normal manufacturing procedures to ensure the quality and consistency of the products.

### Example 1: (Reference)

In this example the plastic, impervious backsheet typically found on a sanitary napkin is replaced by a microporous film {supplied by Exxon Chemical Company under the manufacturing code Exxaire XBF-100W} and positioned directly in contact with the absorbent core.

### Example 2:

In this example in addition to replacing the backsheet with a microprorous film {supplied by Exxon Chemical Company under the manufacturing code Exxaire XBF-100W} as described in example 1 an additional layer has been inserted between the absorbent core and the microporous film. This layer is a one-direction (towards the core) fluid transporting aperatured film made of low Density PE {supplied by Tredegar Corporation, USA under the manufacturing code X-1522}.

**Table 1:**

| Laboratory Assessment of wet-through on returned sanitary napkins. | | |
|---|---|---|
| **Example** | Ave. Load at Failure (grams) | % Pads with Wet-Throuah |
| Example 1 | 6.6 | 22 % |
| Example 2 | 18.5 | < 2 % |

## Claims

1. A disposable absorbent article comprising, a liquid pervious topsheet, an absorbent core and a breathable backsheet, said absorbent core being positioned intermediate said topsheet and said backsheet, said topsheet, core and backsheet each comprising at least one layer, said core comprising a fluid storage layer, said backsheet comprising an outer layer,
each of said layers having a wearer facing surface and a garment facing surface and each of said wearer facing surfaces forming a common interface with an adjacent garment facing surface of an adjacent layer,
said article has a backsheet portion extending from said garment facing surface of said fluid storage layer to said garment facing surface of said outer layer and
wherein at least one garment facing surface in said backsheet portion is separated from an adjacent wearer facing surface by a gas-permeable separating means in the form of a layer such that not more than 50% of the surface of said common interface is in direct contact,
**characterised in that** said outer layer of said backsheet is a microporous 2-dimensional polymeric apertured film, the apertures having average diameters of from 150 micrometers to 5 micrometers.

2. A disposable absorbent article according to claim 1, wherein not more than 45% of the surface of said common interface is in direct contact.

3. A disposable absorbent article according to any of the preceding claims, wherein said separating means comprises apertures having an average diameter of at least 100 micrometers.

4. A disposable absorbent article according to any of the preceding claims, wherein said separating means is a 2-dimensional apertured polymeric film or an apertured formed polymeric film.

5. A disposable article according to any of the preceding claims, wherein said common interface comprises a central portion and a side portion and wherein not more than 50% of said central portion of the surface of said common interface forms a direct contact.

6. A disposable absorbent article according to any of the preceding claims, wherein said article is a sanitary napkin or a panty liner.

## Patentansprüche

1. Absorbierender Wegwerf-Artikel mit einer flüssigkeitsdurchlässigen Deckschicht, einem absorbierenden Kern und einer atmungsaktiven Außenschicht, wobei der absorbierende Kern zwischen der Deckschicht und der Außenschicht angeordnet ist, wobei die Deckschicht, der Kern und die Außenschicht jeweils mindestens eine Lage umfassen, wobei der Kern mindestens eine Fluid-Speicherungs-Lage umfasst, wobei die Außenschicht eine äußere Lage umfasst,
wobei jede der Lagen eine Träger-zugewandte Oberfläche und eine Kleidungsstück-zugewandte Oberfläche aufweist und jede der Träger-zugewandten Oberflächen eine gemeinsame Grenzfläche mit einer benachbarten Kleidungsstück-zugewandten Oberfläche einer benachbarten Lage bildet,
wobei der Artikel einen Außenschicht-Abschnitt aufweist, der von der Kleidungsstück-zugewandten Oberfläche der Fluid-Speicherungs-Lage zu der Kleidungsstück-zugewandten Oberfläche der äußeren Lage verläuft, und
wobei mindestens eine Kleidungsstück-zugewandte Oberfläche in dem Außenschicht-Abschnitt von einer benachbarten Träger-zugewandten Oberfläche durch ein gasdurchlässiges Trennmittel in Form einer Lage derart getrennt ist, dass nicht mehr als 50 % der Oberfläche der gemeinsamen Grenzfläche in direktem Kontakt stehen,
**dadurch gekennzeichnet, dass** die äußere Lage der Außenschicht eine mikroporöse, zweidimensionale, polymere, mit Öffnungen versehene Folie ist, wobei die Öffnungen durchschnittliche Durchmesser von 150 Mikrometer bis 5 Mikrometer aufweisen.

2. Absorbierender Wegwerf-Artikel gemäß Anspruch 1, wobei nicht mehr als 45 % der Oberfläche der gemeinsamen Grenzfläche in direktem Kontakt stehen.

3. Absorbierender Wegwerf-Artikel gemäß einem der vorherigen Ansprüche, wobei das Trennmittel Öffnungen aufweist, die einen durchschnittlichen Durchmesser von mindestens 100 Mikrometer aufweisen.

4. Absorbierender Wegwerf-Artikel gemäß einem der vorherigen Ansprüche, wobei das Trennmittel eine zweidimensionale, mit Öffnungen versehene, polymere Folie oder eine mit Öffnungen versehene, geformte polymere Folie ist.

5. Absorbierender Wegwerf-Artikel gemäß einem der vorherigen Ansprüche, wobei die gemeinsame Grenzfläche einen zentralen Abschnitt und einen Seitenabschnitt umfasst, und wobei nicht mehr als 50 % des zentralen Abschnitts der Oberfläche der gemeinsamen Grenzfläche einen direkten Kontakt bilden.

6. Absorbierender Wegwerf-Artikel gemäß einem der vorherigen Ansprüche, wobei der Artikel eine Damenbinde oder eine Slipeinlage ist.

## Revendications

1. Article absorbant jetable comprenant: une feuille de dessus perméable aux liquides, une âme absorbante et une feuille de fond pouvant respirer, ladite âme absorbante étant placée entre ladite feuille de dessus et ladite feuille de fond, lesdites feuille de dessus, âme et feuille de fond comprenant chacune au moins une couche, ladite âme comprenant une couche de stockage de fluide, ladite feuille de fond comprenant une couche extérieure,
chacune desdites couches présentant une surface tournée vers l'utilisateur et une surface tournée vers le vêtement, et chacune desdites surfaces tournées vers l'utilisateur formant une interface commune avec une surface tournée vers le vêtement, adjacente, d'une couche adjacente,
ledit article présente une partie de feuille de fond s'étendant de ladite surface tournée vers le vêtement de ladite couche de stockage de fluide à ladite surface tournée vers le vêtement de ladite couche extérieure, et
dans lequel au moins une surface tournée vers le vêtement située dans ladite partie de feuille de fond est séparée d'une surface tournée vers l'utilisateur, adjacente, par un moyen de séparation perméable aux gaz se présentant sous la forme d'une couche, de sorte que pas plus de 50% de la surface de ladite interface commune soit en contact direct,
**caractérisé en ce que** ladite couche extérieure de ladite feuille de fond est un film polymère microporeux à deux dimensions, perforé, les orifices ayant des diamètres moyens de 150 µm à 5µm.

2. Article absorbant jetable selon la revendication 1, dans lequel pas plus de 45% de la surface de ladite interface commune est en contact direct.

3. Article absorbant jetable selon l'une quelconque des revendications précédentes, dans lequel ledit moyen de séparation comprend des orifices ayant un diamètre moyen d'au moins 100 µm.

4. Article absorbant jetable selon l'une quelconque des revendications précédentes, dans lequel ledit moyen de séparation est un film polymère à deux dimensions perforé ou un film polymère formé perforé.

5. Article absorbant jetable selon l'une quelconque des revendications précédentes, dans lequel ladite interface commune comprend une partie centrale et une partie latérale, et dans lequel pas plus de 50% de ladite partie centrale de la surface de ladite interface commune forme un contact direct.

6. Article absorbant jetable selon l'une quelconque des revendications précédentes, dans lequel ledit article est une serviette hygiénique ou une garniture de culotte.
